# EUROPEAN PATENT SPECIFICATION

(11) **EP 2 964 158 B1**
(45) Date of publication and mention of the grant of the patent: **12.06.2024**
(21) Application number: 14760154.6
(22) Date of filing: 05.03.2014
(51) Int. Cl.: A61F 2/44, A61F 2/30, A61F 2/46

(54) **LOW PROFILE PLATE**
PLATTE MIT NIEDRIGEM PROFIL
PLAQUE À BAS PROFIL

(30) Priority: 05.03.2013 US 201313785434; 26.02.2014 US 201414190948
(43) Date of publication of application: 13.01.2016
(73) Proprietor: Globus Medical, Inc., Audubon, PA 19403 (US)
(72) Inventor: LAWSON, Stephan, Upper Darby, Pennsylvania 19082 (US); PETERSHEIM, Samuel, Elverson, Pennsylvania 19520 (US); CIANFRANI, Jason, East Norriton, Pennsylvania 19401 (US)
(74) Representative: Morabito, Sara
(86) International application number: PCT/US2014/020928
(87) International publication number: WO 2014/138311

(56) References cited:
- WO-A1-00/66011
- US-A1- 2008 249 569
- US-A1- 2010 145 459
- US-A1- 2010 145 459
- US-A1- 2010 305 704
- US-A1- 2010 312 346
- US-A1- 2012 078 373
- US-A1- 2012 130 495
- US-A1- 2012 277 873

## Description

### CROSS-REFERENCE TO RELATED APPLICATIONS

### FIELD OF THE INVENTION

The present application is generally directed to orthopedic systems, and in particular, to systems including plates and spacers.

### BACKGROUND

Spinal discs and/or vertebral bodies of a spine can be displaced or damaged due to trauma, disease, degenerative defects, or wear over an extended period of time. One result of this displacement or damage may be chronic back pain. In some cases, to alleviate back pain, the disc can be removed and replaced with an implant, such as a spacer, that promotes fusion. In addition to providing one or more spacers, a plating system can be used to further stabilize the spine during the fusion process. Such a plating system can include one or more plates and screws for aligning and holding vertebrae in a fixed position with respect to one another.

US2010/145459 and US2010/312346 disclose spinal systems according to the known art. Accordingly, there is a need for improved systems involving plating systems and spacers for spinal fusion and stabilization.

### SUMMARY OF THE INVENTION

The invention is defined by a spinal system according to independent claim. Further advantageous embodiments of the invention are set forth in the dependent claims.

Various systems, devices and methods (not claimed) related to plating systems are provided. The spinal system comprises a spacer for inserting into an intervertebral space and a plate configured to abut the spacer. The spacer can include an upper surface, a lower surface and an opening that extends between the upper surface to the lower surface, wherein the spacer further includes a tapered leading end. The plate for abutting the spacer can include a plate body, a first opening formed in the plate body for receiving a first bone screw, a second opening formed in the plate body for receiving a second bone screw, a set screw, and a pair of extensions that extend from the plate body that are configured to engage the spacer. The first opening can angled in an upward direction, while the second opening can be angled in a downward direction. The set screw can be configured to prevent back-out of both the first and the second bone screws, wherein the set screw has a first position whereby the first and second bone screws can be inserted past the set screw and into the first and second openings and a second position following rotation of the set screw whereby the first and second bone screws are prevented from backing out by the set screw. A first bone screw is provided for inserting into the first opening in the plate body, wherein the first bone screw is configured to be inserted into a first vertebral body. A second bone screw is provided for inserting into the second opening in the plate body, wherein the second bone screw is configured to be inserted into a second vertebral body different from the vertebral body.

The spinal system comprises a spacer for inserting into an intervertebral space and a plate configured to abut the spacer. The spacer can include an upper surface, a lower surface and an opening that extends between the upper surface to the lower surface, wherein the spacer further includes a concave leading end. The plate for abutting the spacer can include a plate body, a first opening formed in the plate body for receiving a first bone screw, a second opening formed in the plate body for receiving a second bone screw, a set screw, and a pair of extensions that extend from the plate body that are configured to engage the spacer. The first opening can angled in an upward direction, while the second opening can be angled in a downward direction. The set screw can be configured to prevent back-out of at least one of the first and the second bone screws, wherein the set screw has a first position whereby at least one of the first and second bone screws can be inserted past the set screw and into at least one of the first and second openings and a second position following rotation of the set screw whereby at least one of the first and second bone screws are prevented from backing out by the set screw. Each of the pair of extensions can include a window that extends along a length of the extension. A first bone screw is provided for inserting into the first opening in the plate body, wherein the first bone screw is configured to be inserted into a first vertebral body. A second bone screw is provided for inserting into the second opening in the plate body, wherein the second bone screw is configured to be inserted into a second vertebral body different from the vertebral body.

The spinal system comprises a spacer for inserting into an intervertebral space and a plate configured to abut the spacer. The spacer can include an upper surface, a lower surface and an opening that extends between the upper surface to the lower surface. The plate for abutting the spacer can include a plate body, a first opening formed in the plate body for receiving a first bone screw, a second opening formed in the plate body for receiving a second bone screw, a set screw, and a pair of extensions that extend from the plate body that are configured to engage the spacer. The first opening can angled in an upward direction, while the second opening can be angled in a downward direction. The set screw can be configured to prevent back-out of at least one of the first and the second bone screws, wherein the set screw has a first position whereby at least one of the first and second bone screws can be inserted past the set screw and into at least one of the first and second openings and a second position following rotation of the set screw whereby at least one of the first and second bone screws are prevented from backing out by the set screw. Each of the pair of extensions can include a window that extends along a length of the extension. A first bone screw is provided for inserting into the first opening in the plate body, wherein the first bone screw is configured to be inserted into a first vertebral body. A second bone screw is provided for inserting into the second opening in the plate body, wherein the second bone screw is configured to be inserted into a second vertebral body different from the vertebral body. The spacer and the plate are independent from one another such that the spacer can be inserted into a desired spinal location prior to abutting the spacer with the plate.

In other embodiments a spinal system comprises a spacer for inserting into an intervertebral space including an upper surface a lower surface and an opening that extends between the upper surface to the lower surface wherein the spacer further includes a tapered leading end; a plate for abutting the spacer including a plate body, a first opening formed in the plate body for receiving a first bone screw and the first opening is angled in an upward direction, a second opening formed in the plate body for receiving a second bone screw, wherein the second opening is angled in a downward direction; a set screw for preventing back-out of both the first and the second bone screws, having a first position whereby the first and second bone screws can be inserted past the set screw and into the first and second openings and a second position following rotation of the set screw whereby the first and second bone screws are prevented from backing out by the set screw; a pair of extensions extending from the plate body and configured to engage the spacer; a first bone screw for inserting into the first opening in the plate body, configured to be inserted into a first vertebral body, and a second bone screw for inserting into the second opening in the plate body, configured to be inserted into a second vertebral body different from the vertebral body.

Preferably in the above embodiments the spacer is a C-shaped spacer.

Preferably in the above embodiments the spacer comprises an upper chamfer and a lower chamfer.

Preferably in the above embodiments the pair of extensions that extend from the plate body extend around the outer surface of the spacer, more preferably also include inward protrusions for inserting into outer sidewalls of the spacer and optionally the spacer includes a pair of notches that correspond to the pair of extension of the plate to form a first locking mechanism between the plate and the spacer.

Preferably in the above embodiments the opening in the spacer is bounded by at least one convex surface.

Preferably in the above embodiments the pair of extensions that extend from the plate body extend within the outer surface of the spacer.

In other embodiments a spinal system comprises a spacer for inserting into an intervertebral space including an upper surface, a lower surface, and an opening that extends between the upper surface to the lower surface, wherein the spacer further includes a concave leading end; a plate for abutting the spacer including a plate body, a first opening formed in the plate body for receiving a first bone screw, wherein the first opening is angled in an upward direction and a second opening formed in the plate body for receiving a second bone screw, wherein the second opening is angled in a downward direction; a set screw for preventing back-out of at least one of the first and the second bone screws, wherein the set screw has a first position whereby at least one of the first and second bone screws can be inserted past the set screw and into at least one of the first and second openings and a second position following rotation of the set screw whereby at least one of the first and second bone screws is prevented from backing out by the set screw; a pair of extensions that extend from the plate body configured to engage the spacer, and wherein each extension includes a window that extends along a length of the extension; a first bone screw for inserting into the first opening in the plate body, configured to be inserted into a first vertebral body; and a second bone screw for inserting into the second opening in the plate body, configured to be inserted into a second vertebral body different from the vertebral body.

Preferably in these embodiments the pair of extensions of the plate comprise lateral extensions that engage an outer surface of the spacer.

Preferably in the above embodiments the pair of extensions of the plate comprise inward protrusions that extend into notches formed on an outer surface of the spacer.

Preferably in the above embodiments the set screw is configured to prevent back-out of both the first and the second bone screws in the plate and more preferably upon rotation of the set screw, the set screw abuts side surfaces of heads of the first and second bone screws.

Preferably in the above embodiments the leading end of the spacer is tapered.

In other embodiments a spinal system comprises spinal system with a spacer for inserting into an intervertebral space including an upper surface and a lower surface, and an opening that extends between the upper surface to the lower surface; a plate for abutting the spacer including a plate body and a first opening formed in the plate body for receiving a first bone screw, wherein the first opening is angled in an upward direction and a second opening formed in the plate body for receiving a second bone screw, wherein the second opening is angled in a downward direction; a pair of extensions that extend from the plate body configured to engage the spacer and wherein each extension includes a window that extends along a length of the extension; a first bone screw for inserting into the first opening in the plate body configured to be inserted into a first vertebral body; a second bone screw for inserting into the second opening in the plate body configured to be inserted into a second vertebral body different from the vertebral body. In said embodiments the spacer and the plate are independent from one another such that the spacer can be inserted into a desired spinal location prior to abutting the spacer with the plate.

Preferably in the above embodiments the spacer comprises an allograft spacer or a PEEK spacer. More preferably the allograft spacer includes at least one chamfer for accommodating either the first bone screw or the second bone screw.

Preferably in the above embodiments the pair of extensions each include a window that receives a hump portion of the spacer to create a locking mechanism between the plate and the spacer.

### BRIEF DESCRIPTION OF THE DRAWINGS

FIGS. 1A-1D illustrate different views of a low profile plate attached to a spacer according to some embodiments.
FIGS. 2A-2D illustrate different views of the low profile plate shown in FIGS. 1A-1D.
FIGS. 3A-3D illustrate different views of a PEEK spacer to be used with the low profile plate shown in FIGS. 2A-2D.
FIGS. 4A-4D illustrate different views of an allograft spacer to be used with the low profile plate shown in FIGS. 2A-2D.
FIGS. 5A-5D illustrate different views of a second alternative embodiment of a low profile plate attached to a spacer according to some embodiments.
FIGS. 6A-6D illustrate different views of the low profile plate shown in FIGS. 5A-5D.
FIGS. 7A-7D illustrate different views of a PEEK spacer to be used with the low profile plate in FIGS. 6A-6D.
FIGS. 8A-8D illustrate different views of an allograft spacer to be used with the low profile plate in FIGS. 6A-6D.
FIGS. 9A-9D illustrate different views of a third alternative embodiment of a low profile plate attached to a spacer according to some embodiments.
FIGS. 10A-10D illustrate different views of the low profile plate shown in FIGS. 9A-9D.
FIGS. 11A-11D illustrate different views of a fourth alternative embodiment of a low profile plate attached to a spacer according to some embodiments.
FIGS. 12A-12D illustrate different views of the low profile plate shown in FIGS. 11A 11D.
FIGS. 13A-13D illustrate different views of a multi-piece allograft spacer to be used with the low profile plates discussed above according to some embodiments.
FIGS. 14A-14D illustrate different views of an alternative multi-piece allograft spacer to be used with the lower profile plates discussed above according to some embodiments.

### DETAILED DESCRIPTION OF THE ILLUSTRATED EMBODIMENTS

The present application is generally directed to orthopedic systems, and in particular, to systems including plates and spacers.

The present application discloses orthopedic plating systems that can be used in spinal surgeries, such as spinal fusions. The plating systems disclosed herein include a plate and a spacer that are independent from one another. In some cases, the plate and the spacer can be preattached to one another before positioning them in a desired location of the spine. In other cases, the spacer can first be inserted into a desired location of the spine, and then the plate can be inserted thereafter. Advantageously, the plating systems disclosed herein are of low-profile. For example, they can provide a very small, anterior footprint cervical plate solution for fusion procedures. One skilled in the art will appreciate that while the plating systems can be used with cervical procedures, the plating systems are not limited to such areas, and can be used with other regions of the spine.

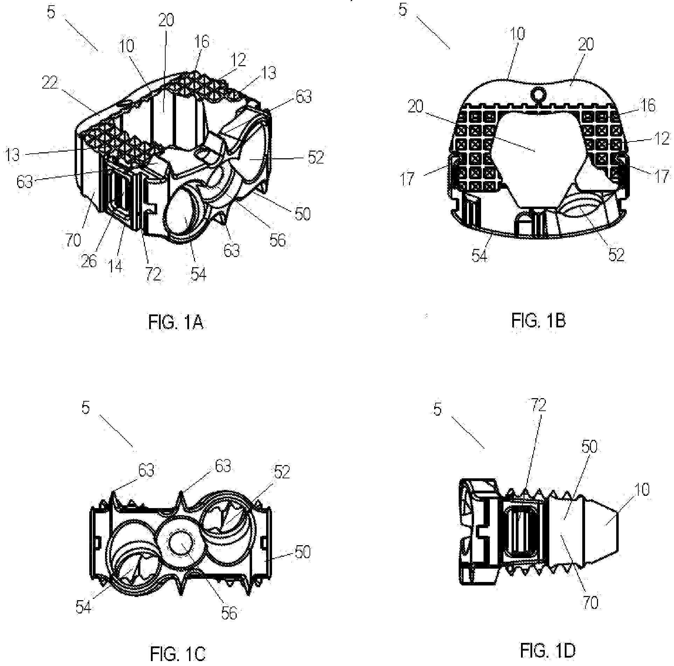

FIGS. 1A-1D illustrate different views of a plating system comprising a low profile plate attached to a spacer according to some embodiments. The plating system 5 includes a spacer 10 attached to a low-profile plate 50. Advantageously, the plating system 5 can be inserted through an anterior approach into a spine, and can desirably provide a small anterior footprint.

The spacer 10 is configured to have an upper surface 12, a lower surface 14, and a leading end 22. In some embodiments, the upper surface 12 and/or lower surface 14 includes texturing 16, such as teeth, ribs, ripples, etc. to assist in providing frictional contact with adjacent vertebral bodies. In some embodiments, the leading end 22 of the spacer 10 can be slightly tapered, as shown in FIG. 1A. With the taper, the leading end 22 can serve as a distraction surface that helps the spacer to be inserted into an intervertebral space. As shown in FIG. IB, the leading end 22 can be concave, though in other embodiments, the leading end 22 can be straight or convex.

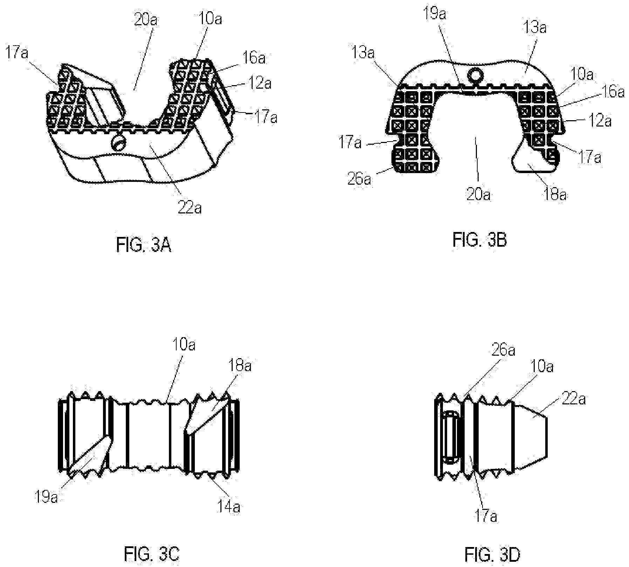

The spacer 10 can be substantially C-shaped (as shown in FIG. 3B), whereby it includes two side arms 13 that surround an inner opening 20. Adjacent the side arms 13 is a convex wall 19. In some embodiments, the convex wall 19 is substantially parallel to the concave surface of the leading end 22. The opening 20, which is configured to receive natural or synthetic graft material therein to assist in a fusion procedure, has an open side that is opposite convex wall 19, thereby giving the spacer 10 its C-shape.

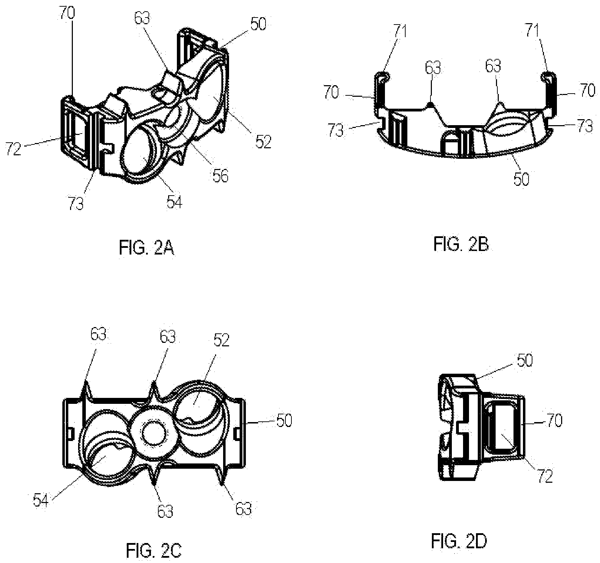

The spacer 10 has a number of unique features that accommodate the attachment of a plate 50 thereto. Each of the side arms 13 of the spacer 10 includes a notch 17 (shown in FIG. 3B) for receiving a corresponding protrusion 71 of a lateral arm or extension 70 of the plate 50, thereby advantageously forming a first locking mechanism between the spacer 10 and the plate 50. In addition, in some embodiments, each of the side arms 13 of the spacer 10 can also include a hump region 26 (shown in FIG. 3B) that can extend in part into a window 72 of an attached plate 50 (shown in FIG. 2 A), thereby advantageously providing a second locking mechanism between the spacer 10 and the plate 50. Advantageously, by providing secure first and second locking mechanisms between the spacer 10 and the plate 50, the plate and spacer will be kept securely together during any type of impaction of the plating system within the body. Furthermore, each of the side arms 13 of the spacer 10 can include a cut-away portion or chamfer 18, 19 (shown in FIG. 3C) to advantageously accommodate screws which pass through the plate. In embodiments that involve a pair of screws through the plate 50 - one of which passes in an upward direction, and the other of which passes in a downward direction - one side arm 13 of the spacer 10 will include an upper chamfer 18 formed on an upper surface to accommodate the upwardly directed screw, while the second side arm 13 of the spacer will include a lower chamfer 19 formed on a lower surface to accommodate the downwardly directed screw.

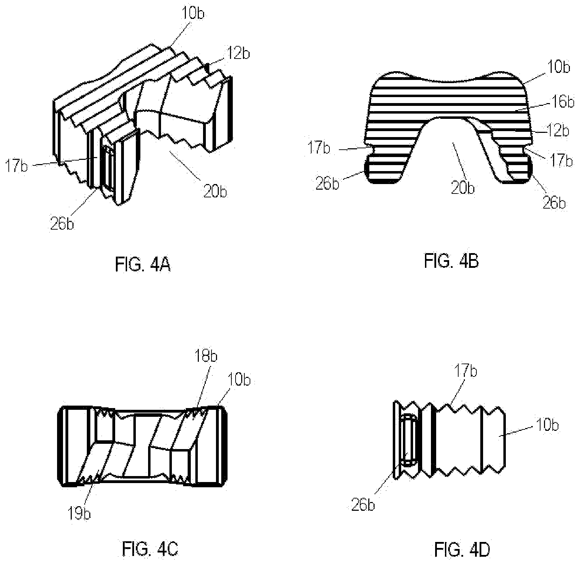

The spacer 10 can be formed of any material. In some embodiments, the spacer 10 is formed of a polymer, such as PEEK, as shown in FIG. 3 A. In some embodiments, the spacer 10 is formed of allograft bone, as shown in FIG. 4A. In some instances, to form an allograft implant, allograft bone may be cut or shaved from a desired bone member. The cut allograft bone will then be assembled together, using an adhesive or mechanical fastener (e.g., bone pins). Accordingly, in some embodiments, an allograft spacer 10 is formed of two, three, four or more layers that are assembled together, such as by one or more bone pins. One particular advantage of the invention is that the plate 50 can work with a variety of different spacers 10, as the plate 50 is independently removable from and attachable to the spacer 10. Regardless of whether a surgeon chooses to implant an allograft spacer or PEEK spacer 10 into an intervertebral space, the surgeon can simply attach the low-profile plate 50 to the spacer 10 following implantation into the intervertebral space.

The plate 50 is configured to have a plate body and a pair of lateral extensions 70 that extend from the plate body, each of which has a protrusion 71, for inserting into a corresponding notch 17 of the spacer 10. These lateral extensions 70 help form the first locking mechanism between the plate 50 and the spacer 10, as discussed above. In addition, the lateral extensions 70 of the plate 50 each include a window 72 (shown in FIG. 2A) for receiving a hump region 26 on the arms 17 of the spacer 10, thereby helping to form the second locking mechanism between the plate 50 and the spacer 10, as discussed above.

In addition to attaching to the spacer 10, the plate 50 is also configured to attach into one or more vertebral bodies via one or more bone screws. As shown in FIG. 1A, the plate 50 includes a first screw hole 52 and a second screw hole 54 for receiving bone screws therein. In some embodiments, screw hole 52 is angled upwardly such that an inserted bone screw passes upward into an upper vertebral body, while screw hole 54 is angled downwardly such that an inserted bone screw passes downward into a lower vertebral body. While the illustrated embodiment illustrates a pair of screw holes for receiving a pair of bone screws, it is possible to have one, three, four, five or more screw holes for receiving a different number of bone screws.

Over time, it is possible for bone screws to back-out. The plate 50 thus has a blocking or set screw 56 (shown in FIG. 1C) that assists in preventing back-out of inserted bone screws. As shown in FIG. 1C, the set screw 56 can be in an initial position that allows first and second bone screws to pass through holes 52, 54. Once the bone screws have been inserted through the holes 52, 54, the set screw 56 can be rotated (e.g., 90 degrees), to thereby block the bone screws and prevent back out of the bone screws. In some embodiments, the set screw 56 abuts a side of the head of the bone screws to prevent back-out of the bone screws, while in other embodiments, the set screw 56 rests over a top of the head of the bone screws to prevent back-out of the bone screws. In some embodiments, the set screw 56 comes pre-fixed with the plate 50. As shown in FIG. 1C, a single set screw 56 can be used to conveniently block a pair of bone screws. In other embodiments, each bone screw can be assigned its own set screw, which can operate independently of one another, to prevent back-out of the bone screw.

The plate 50 can also include one or more knife-like edges 63 that provide additional torsional stabilization when the plate 50 rests against a bone member. As shown in FIG. 1C, the knife-like edges 63 can be formed on both the upper and lower surfaces of the plate 50 body. While the illustrated embodiment shows a pair of knife-like edges 63 on an upper surface of the plate body and a pair of knife-like edges 63 on a lower surface of the plate body, one skilled in the art will appreciate that a different number of knife-like edges 63 can be provided.

FIGS. 2A-2D illustrate different views of the low profile plate shown in FIGS. 1A-1D. From these views, one can see the pair of lateral extensions 70 that extend from the body of the plate 50. At the distal end of each of the lateral extensions 70 is an inwardly-facing protrusion 71 that is configured to fit into a corresponding notch in the spacer 10. In addition, from these views, one can see the windows 72 that are formed in each of the lateral extensions 70. The windows 72 advantageously receive hump regions 26 of the spacer to provide a locking mechanism, and also help to improve desirable radiolucency. Advantageously, the windows 72 can have rounded edges to accommodate the spacer 10 therein. While the illustrated windows 72 are shown as rectangular with rounded edges, in other embodiments, the windows 72 can have a different shape, such as circular or oval. In some embodiments, the plate 50 is assembled axially to the spacer 10.

In some embodiments, the low profile plate 50 can also include indented gripping sections 73 (shown in FIGS. 2A and 2B). These indented gripping sections 73 advantageously provide a gripping surface for an insertion instrument, thereby facilitating easy delivery of the plate to a spacer body during surgery.

FIGS. 3A-3D illustrate different views of a PEEK spacer to be used with the low profile plate shown in FIGS. 2A-2D. From these views, one can see how the spacer 10a includes an upper surface 12a and a lower surface 14a with texturing 16a; a generally C-shaped body including a pair of arms 13a each having a notch 17a formed therein and an upper chamfer 18a or lower chamfer 19a; and a tapered leading edge 22a. In addition, one skilled in the art can appreciate the substantially symmetric shape of the inner opening 20a, which serves as a graft hole for receiving graft material therein.

FIGS. 4A-4D illustrate different views of an allograft spacer to be used with the lower profile plate shown in FIGS. 2A-2D. While the allograft spacer 10b shares similar features to the PEEK spacer 10a shown in previous figures, such as the notches 17b, hump surfaces 26b, and chamfers 18b, 19b, the allograft spacer 10b need not be the same. For example, the shape of the graft opening 20b can be more of an arch, as shown in FIG. 4B.

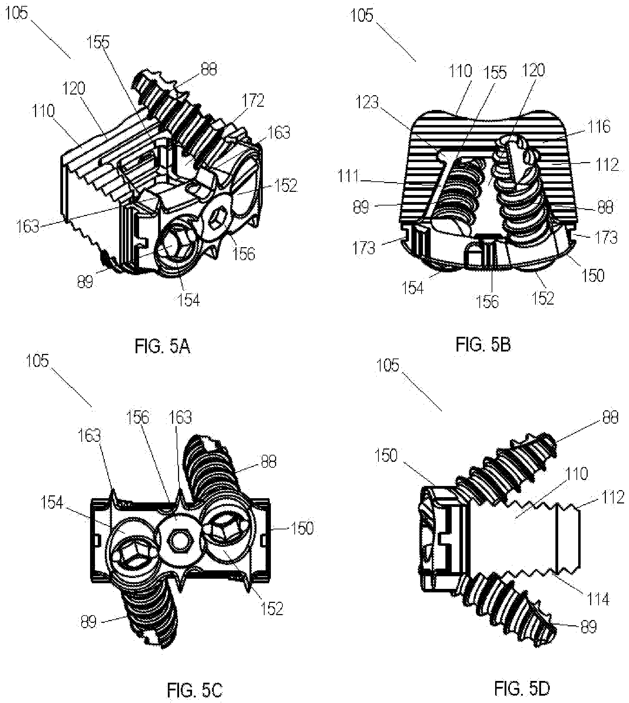

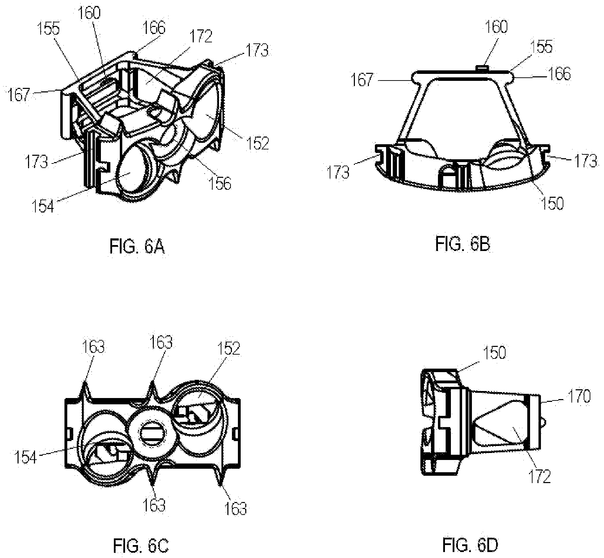

FIGS. 5A-5D illustrate different views of a second alternative embodiment of a low profile plate attached to a spacer according to some embodiments. Rather than having a plate 50 with lateral extensions 70 that extend around the outer surface of a spacer 10, the present embodiment of the plating system 105 includes a plate 150 with an enclosed posterior extension 155 that fits within the body of the spacer 110. The enclosed posterior extension 155 includes extending surfaces 166, 167 that are fitted into corresponding inlets 121 , 123 formed in the body of the spacer 120, thereby forming a first locking mechanism between the plate 150 and the spacer 110. In addition, the enclosed posterior extension 155 of the plate 50 includes one or more deformable locking tabs 160 (shown in FIG. 6B) that securely lock into tab holes 181a in the spacer body 110, thereby forming a second locking mechanism between the plate 150 and the spacer 110. While in some embodiments, the plate 150 can be attached to the spacer 110 after inserting the spacer 110 into a desired location in the body, in other embodiments, the plate 150 can be pre-assembled with the spacer 110 prior to inserting the plating system 105 into the desired location.

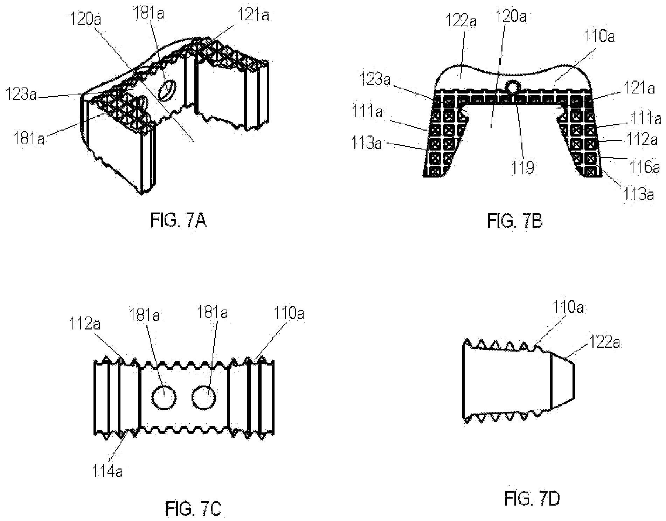

Like the spacer 10 in FIG. 1 A, the spacer 110 is configured to have an upper surface 112, a lower surface 114, and a leading end 122. In some embodiments, the upper surface 112 and/or lower surface 114 includes texturing 116, such as teeth, ribs, ripples, etc. to assist in providing frictional contact with adjacent vertebral bodies. In some embodiments, the leading end 122 of the spacer 110 can be slightly tapered, as shown in FIG. 7D. With the taper, the leading end 122 can serve as a distraction surface that helps the spacer 110 to be inserted into an intervertebral space. As shown in FIG. IB, the leading end 122 can be concave, though in other embodiments, the leading end 122 can be straight or convex.

The spacer 110 can be substantially C-shaped (as shown in FIG. 7B), whereby it includes two side arms 113 that surround an inner opening 120. Adjacent the side arms 113 is a straight wall 119 that forms the border of the graft opening 120. The straight wall 119 can include one or more tab holes 181 (shown in FIG. 7A) for receiving deformable tab locks 160 therein. The graft opening 20, which is configured to receive natural or synthetic graft material therein to assist in a fusion procedure, has an open side that is opposite the straight wall 119, thereby giving the spacer 110 its C-shape.

In some embodiments, the graft opening 120 (shown in FIG. 7B) has a different shape from the opening 20 of the spacer 10 of the prior embodiment, as the graft opening 120 is configured to not only receive graft material, but also the enclosed posterior extension 155 of the plate 150. For example, the graft opening 120 includes two inlets - a first inlet 121 formed at the junction between the first arm 113 and wall 119 and a second inlet 123 formed at the junction between the second arm 113 and wall 119 (shown in FIG. 7B) - for receiving outwardly extending surfaces 166, 167 of the plate 150 (shown in FIG. 6B). In addition, the graft opening 120 includes two outwardly tapering walls 111 that provide enough space to accommodate any bone screws inserted in the plate 150. As such, additional chamfers 18, 19 (as shown in FIG. 3B) are optional.

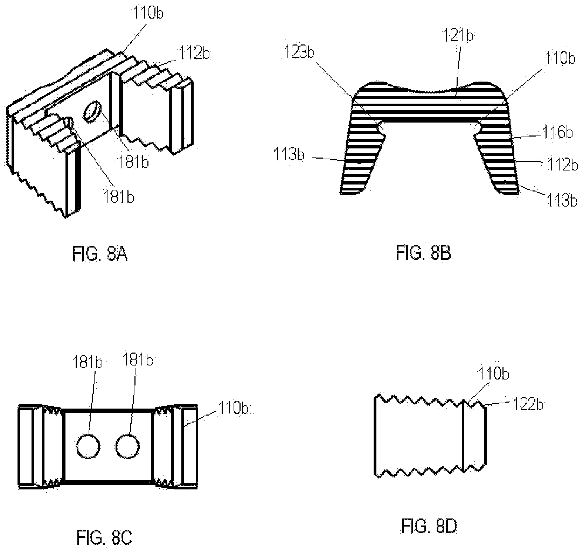

Like spacer 10, the spacer 110 can be formed of a variety of materials. In some embodiments, the spacer 110 comprises PEEK, as shown in FIG. 7A, while in other embodiments, the spacer 110 comprises allograft bone, as shown in FIG. 8A.

The plate 150 is configured to have a plate body, and an enclosed posterior extension 155 that extends from the plate body, which is received within and retains the spacer 110. The enclosed posterior extension 155 includes first and second outwardly extending surfaces 166, 167 that fit into inlets 121, 123 formed within the spacer 110 body to form a first locking mechanism. In addition, one or more deformable tab locks 160 extend from an exterior surface of the enclosed posterior extension 155 and are received in corresponding tab holes 181 in the spacer 150 to form a second locking mechanism. In some embodiments, the side walls of the enclosed posterior extension 155 can include one or more windows 172 (shown in FIG. 6A) for improving radiolucency of the plating system. In some embodiments, the plate 150 is assembled axially to the spacer 110.

In addition to attaching to the spacer 110, the plate 150 is also configured to attach into one or more vertebral bodies via one or more bone screws 88, 89. As shown in FIG. 5A, the plate 150 includes a first screw hole 152 and a second screw hole 154 for receiving bone screws 88, 89 therein. In some embodiments, screw hole 152 is angled upwardly such that an inserted bone screw 88 passes upward into an upper vertebral body, while screw hole 154 is angled downwardly such that an inserted bone screw 89 passes downward into a lower vertebral body. While the illustrated embodiment illustrates a pair of screw holes for receiving a pair of bone screws, it is possible to have one, three, four, five or more screw holes for receiving a different number of bone screws.

Over time, it is possible for bone screws to back-out. The plate 150 thus has a blocking or set screw 156 (shown in FIG. 5C) that assists in preventing back-out of inserted bone screws. As shown in FIG. 5C, the set screw 156 can be in an initial position that allows first and second bone screws to pass through holes 152, 154. Once the bone screws have been inserted through the holes 152, 154, the set screw 156 can be rotated (e.g., 90 degrees), to thereby block the bone screws and prevent back out of the bone screws. In some embodiments, the set screw 156 abuts a side of the head of the bone screws to prevent back-out of the bone screws, while in other embodiments, the set screw 156 rests over a top of the head of the bone screws to prevent back-out of the bone screws. In some embodiments, the set screw 156 comes pre-fixed with the plate 150. As shown in FIG. 5C, a single set screw 16 can be used to conveniently block a pair of bone screws. In other embodiments, each bone screw can be assigned its own set screw, which can operate independently of one another, to prevent back-out of the bone screw.

The plate 150 can also include one or more knife-like edges 163 that provide additional torsional stabilization when the plate 150 rests against a bone member. As shown in FIG. 5C, the knife-like edges 163 can be formed on both the upper and lower surfaces of the plate 150 body. While the illustrated embodiment shows a pair of knife-like edges 163 on an upper surface of the plate body and a pair of knife-like edges 163 on a lower surface of the plate body, one skilled in the art will appreciate that a different number of knife-like edges 163 can be provided.

FIGS. 6A-6D illustrate different views of the low profile plate shown in FIGS. 5A-5D. From these views, one can see the enclosed posterior extension 155 that extends from the body of the plate 150. At the distal end of the enclosed posterior extension 155 are a pair of outwardly extending surfaces 166, 167 that are configured to fit within inlets 121, 123 formed in the spacer. From these views, one can also see the deformable tab lock 160 (FIG. 6B) that can help secure the plate 150 to the spacer 110. In addition, from these views, one can see the windows 172 that are formed in each of the arms of the enclosed posterior extension 155. The windows 172 advantageously help to improve desirable radiolucency, and are of large size to provide a large viewing surface area. While the illustrated windows 172 are shown as triangular with rounded edges, in other embodiments, the windows 172 can have a different shape, such as circular or oval. In some embodiments, the plate 150 is assembled axially to the spacer 110.

In some embodiments, the low profile plate 150 can also include indented gripping sections 173 (shown in FIGS. 6A and 6B). These indented gripping sections 173 advantageously provide a gripping surface for an insertion instrument, thereby facilitating easy delivery of the plate to a spacer body during surgery.

FIGS. 7A-7D illustrate different views of a PEEK spacer to be used with the low profile plate shown in FIGS. 5A-5D. From these views, one can see how the spacer 110a includes an upper surface 112a and a lower surface 114a with texturing 116a; a generally C-shaped body including a pair of arms 113a each having an inner inlet 121, 123a formed therein; and a tapered leading edge 122a. In addition, one skilled in the art can appreciate the substantially symmetric shape of the inner opening 120a, which serves as a graft hole for receiving graft material therein.

FIGS. 8A-8D illustrate different views of an allograft spacer to be used with the lower profile plate shown in FIGS. 5A-5D. While the allograft spacer 110b shares similar features to the PEEK spacer 110a shown in previous figures, such as the C-shaped body including a pair of arms 113b each having an inlet 121b, 123b formed therein, the allograft spacer 110b need not be the same.

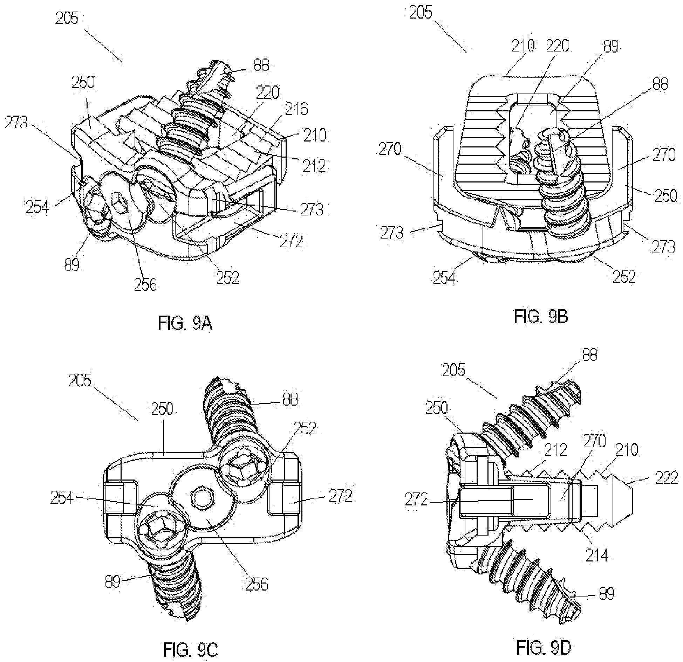

FIGS. 9A-9D illustrate different views of a third alternative embodiment of a low profile plate attached to a spacer according to some embodiments. In the present embodiment, the plating system 205 includes a plate 250 having lateral arms or extensions 270 that extend around an exterior surface of a spacer 210. The lateral extensions 270 extend wider than the lateral extensions 70 in the first embodiment, and do not necessarily have to interlock with the spacer 210. While in some embodiments, the plate 250 can be attached to the spacer 210 after inserting the spacer 210 into a desired location in the body, in other embodiments, the plate 250 can be pre-assembled with the spacer 210 prior to inserting the plating system 205 into the desired location.

Like the spacer 10 in FIG. 1A, the spacer 210 is configured to have an upper surface 212, a lower surface 214, and a leading end 222. In some embodiments, the upper surface 212 and/or lower surface 214 includes texturing 216, such as teeth, ribs, ripples, etc. to assist in providing frictional contact with adjacent vertebral bodies. In some embodiments, the leading end 222 of the spacer 210 can be slightly tapered, as shown in FIG. 9D. With the taper, the leading end 222 can serve as a distraction surface that helps the spacer 210 to be inserted into an intervertebral space. As shown in FIG. 9B, the leading end 222 can be slightly concave, though in other embodiments, the leading end 122 can be straight or convex. Unlike previously illustrated spacers, the spacer 210 can have a graft hole 220 that is completely enclosed. As shown in FIG. 9B, the graft hole 220 can surrounded by four walls. In addition, the spacer 210 can include four outer walls: two straight walls, a convex wall and a concave wall.

In some embodiments, the graft opening 220 (shown in FIG. 9B) has a different shape from the openings of prior embodiments, as the graft opening 220 is enclosed. While the graft opening 220 is rectangular with rounded edges, in other embodiments, the graft opening 220 can have a different shape. For example, in some embodiments, the graft opening 220 can have curved walls, instead of straight walls, or can have tapered walls, instead of straight walls.

Like spacer 10, the spacer 210 can be formed of a variety of materials. In some embodiments, the spacer 210 comprises allograft bone, while in other embodiments, the spacer 210 comprises PEEK.

The plate 250 is configured to have a pair of lateral extensions 270 that receive the spacer 220. As shown in FIG. 9A, in some embodiments, the lateral extensions 270 include one or more windows 272 for improving radiolucency of the plating system. In some embodiments, the plate 250 is assembled axially to the spacer 210.

In addition to capturing the spacer 210, the plate 250 is also configured to attach into one or more vertebral bodies via one or more bone screws 88, 89. As shown in FIG. 9A, the plate 250 includes a first screw hole 252 and a second screw hole 254 for receiving bone screws 88, 89 therein. In some embodiments, screw hole 252 is angled upwardly such that an inserted bone screw 88 passes upward into an upper vertebral body, while screw hole 254 is angled downwardly such that an inserted bone screw 89 passes downward into a lower vertebral body. While the illustrated embodiment illustrates a pair of screw holes for receiving a pair of bone screws, it is possible to have one, three, four, five or more screw holes for receiving a different number of bone screws.

Over time, it is possible for bone screws to back-out. The plate 250 thus has a blocking or set screw 256 (shown in FIG. 9C) that assists in preventing back-out of inserted bone screws. As shown in FIG. 9C, the set screw 256 can be in an initial position that allows first and second bone screws to pass through holes 252, 254. Once the bone screws have been inserted through the holes 252, 254, the set screw 256 can be rotated (e.g., 90 degrees), to thereby block the bone screws and prevent back out of the bone screws. In some embodiments, the set screw 256 abuts a side of the head of the bone screws to prevent back-out of the bone screws, while in other embodiments, the set screw 256 rests over a top of the head of the bone screws to prevent back-out of the bone screws. In some embodiments, the set screw 256 comes pre-fixed with the plate 250. As shown in FIG. 9C, a single set screw 256 can be used to conveniently block a pair of bone screws. In other embodiments, each bone screw can be assigned its own set screw, which can operate independently of one another, to prevent back-out of the bone screw.

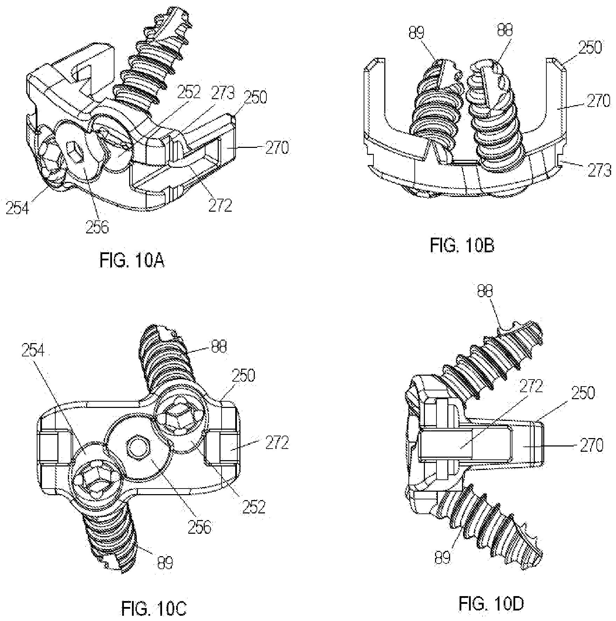

FIGS. 10A-10D illustrate different views of the low profile plate shown in FIGS. 9A-9D. From these views, one can see the lateral extensions 270 that extend from the body of the plate 250. From these views, one can also see the windows 272 (FIG. 10A) that extend along a substantial length of the lateral extensions 270. In some embodiments, each window 272 has a length greater than half the length of each lateral extension 270, thereby advantageously increasing the radiolucency of the plating system. In some embodiments, the plate 250 is assembled axially to the spacer 210.

In some embodiments, the low profile plate 250 can also include indented gripping sections 273 (shown in FIGS. 10A and 10B). These indented gripping sections 273 advantageously provide a gripping surface for an insertion instrument, thereby facilitating easy delivery of the plate to a spacer body during surgery.

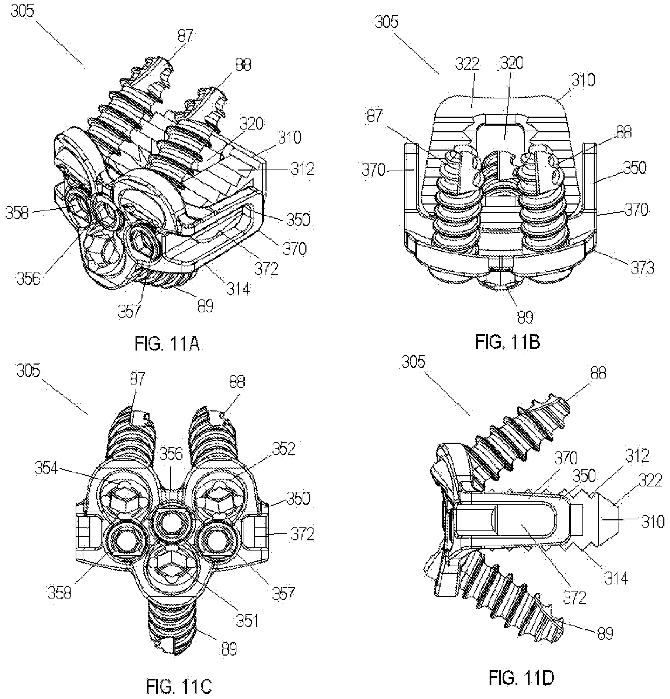

FIGS. 11A-11D illustrate different views of a fourth alternative embodiment of a low profile plate attached to a spacer according to some embodiments. Like the previous embodiment, the plating system 305 includes a plate 350 having lateral arms or extensions 370 that extend around an exterior surface of a spacer 310. The lateral extensions 370 extend wider than the lateral extensions 70 in the first embodiment, and do not necessarily have to interlock with the spacer 310. While in some embodiments, the plate 350 can be attached to the spacer 310 after inserting the spacer 310 into a desired location in the body, in other embodiments, the plate 350 can be pre-assembled with the spacer 310 prior to inserting the plating system 305 into the desired location.

Like the spacer 10 in FIG. 1A, the spacer 310 is configured to have an upper surface 312, a lower surface 314, and a leading end 322. In some embodiments, the upper surface 312 and/or lower surface 314 includes texturing 316, such as teeth, ribs, ripples, etc. to assist in providing frictional contact with adjacent vertebral bodies. In some embodiments, the leading end 322 of the spacer 310 can be slightly tapered, as shown in FIG. 11D. With the taper, the leading end 322 can serve as a distraction surface that helps the spacer 310 to be inserted into an intervertebral space. As shown in FIG. 11 B, the leading end 322 can be slightly concave, though in other embodiments, the leading end 322 can be straight or convex. In some embodiments, the spacer 310 can have a graft hole 320 that is completely enclosed. As shown in FIG. 11B, the graft hole 320 can surrounded by four walls. In addition, the spacer 320 can be comprised of four outer walls: two straight, one concave and one convex.

In some embodiments, the graft opening 320 (shown in FIG. 11B) of the spacer 310 is enclosed. While the graft opening 320 is rectangular with rounded edges, in other embodiments, the graft opening 320 can have a different shape. For example, in some embodiments, the graft opening 320 can have curved walls, instead of straight walls, or can have tapered walls, instead of straight walls.

Like spacer 10, the spacer 310 can be formed of a variety of materials. In some embodiments, the spacer 210 comprises allograft bone, while in other embodiments, the spacer 310 comprises PEEK.

The plate 350 is configured to have a pair of lateral extensions 370 that receive the spacer 320. As shown in FIG. 11A, in some embodiments, the lateral extensions 370 include one or more windows 372 for improving radiolucency of the plating system. In some embodiments, the plate 350 is assembled axially to the spacer 310.

In addition to capturing the spacer 310, the plate 350 is also configured to attach into one or more vertebral bodies via one or more bone screws 88, 89. As shown in FIG. 9A, the plate 350 includes a first screw hole 351, a second screw hole 352 and a third screw hole 354 for receiving bone screws 87, 88, 89 therein. In some embodiments, screw holes 352 and 354 are angled upwardly such that inserted bone screws 87, 88 pass upward into an upper vertebral body, while screw hole 351 is angled downwardly such that inserted bone screw 89 passes downward into a lower vertebral body. While the illustrated embodiment illustrates three screw holes for receiving three bone screws, it is possible to have one, two, four, five or more screw holes for receiving a different number of bone screws.

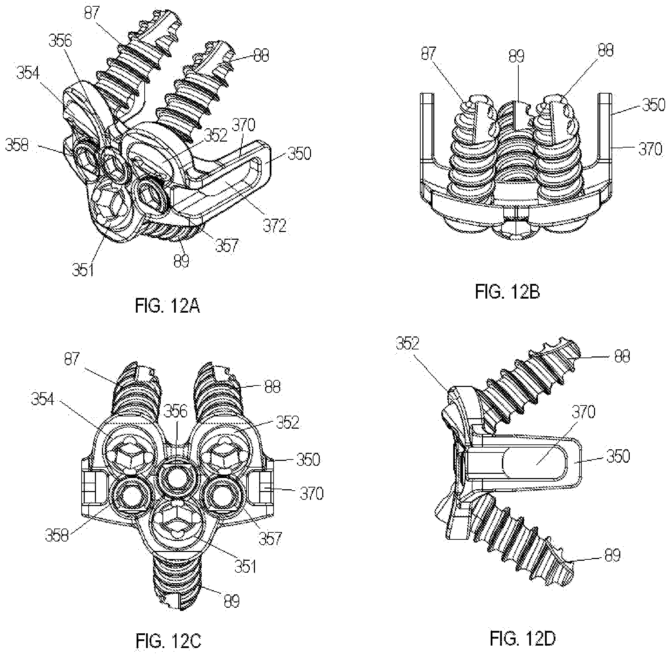

Over time, it is possible for bone screws to back-out. The plate 350 thus has blocking or set screws 356, 357, 358 (shown in FIG. 12C), each of which corresponds to one of screw holes 351, 352, 354. As shown in FIG. 12C, the set screws 356, 357, 358 can be in an initial position that allows first, second and third bone screws to pass through holes 351 , 352, 354. Once the bone screws have been inserted through the holes 351, 352, 354, the set screws 356, 357, 358 can be rotated (e.g., 90 degrees), to thereby block the bone screws and prevent back out of the bone screws. In some embodiments, the set screws 356, 357, 358 abut a side of the head of the bone screws to prevent back-out of the bone screws, while in other embodiments, the set screws 356, 357, 358 rest over a top of the head of the bone screws to prevent back-out of the bone screws. In some embodiments, the set screws 356, 357, 358 come pre-fixed with the plate 350. As shown in FIG. 12C, a single set screw 356, 357, 358 can be used to conveniently block a single bone screws. In other embodiments, each set screw can be designed to block more than one set screw to prevent back-out of the bone screw.

FIGS. 12A-12D illustrate different views of the low profile plate shown in FIGS. 11A-11D. From these views, one can see the lateral extensions 370 that extend from the body of the plate 350. From these views, one can also see the windows 372 (FIG. 12A) that extend along a substantial length of the lateral extensions 370. In some embodiments, each window 372 has a length greater than half the length of each lateral extension 370, thereby advantageously increasing the radiolucency of the plating system. In some embodiments, the plate 350 is assembled axially to the spacer 310.

The plating systems describe include a plate that is independent from a spacer. The plate is low-profile and can be used with any type of spacer, such as allograft or PEEK.

FIGS. 13A-13D illustrate different views of a multi-piece allograft spacer to be used with the low profile plates discussed above according to some embodiments. The multi-piece allograft spacer 410 can be formed of an upper member 436 and a lower member 438 that are connected together via one or more pins 475. The upper member 436 and the lower member 438 each include cut-out portions that help form a graft opening 420 in the spacer 410.

The upper member 436 can include an upper surface having bone engagement surfaces (e.g., ridges, teeth, ribs) and a lower interfacing surface 446. The lower member 438 can include a lower surface having bone engagement surfaces (e.g., ridges, teeth, ribs) and an upper interfacing surface 448. In some embodiments, the upper member 436 can include one or more holes 462, while the lower member 438 can include one or more holes 464 which align with the one or more holes 462 of the upper member. The aligned holes are configured to receive one or more pins 475 to keep the upper and lower members of the allograft spacer together. In some embodiments, the pins 475 are also formed of bone material, such as allograft.

As shown best in FIG. 13C, the lower interfacing surface 446 of the upper member 436 is directly engaged with the upper interfacing surface 448 of the lower member 438. While the lower interfacing surface 446 and the upper interfacing surface 448 can be flat-on-flat, as both surfaces are planar, in some embodiments (as shown in FIG. 13C), the interface between the two surfaces is at an angle relative to the holes for receiving the pins 475. In other words, the pins 475 are received at an angle to the interface between the upper member 436 and the lower member 438. In addition, as shown in FIG. 13C, holes 462 and 464 need not go through the entirety of their respective members. For example, as shown in FIG. 13C, while hole 462 goes entirely through the upper and lower surface of the upper member 436, hole 464 goes only through the upper surface of the lower member 438, and does not go through to the lower surface. Accordingly, in some embodiments, aligned holes 462 and 464 create a "blind" pinhole, whereby the hole does not go through the uppermost and lowermost surfaces of the spacer 410. Advantageously, in some embodiments, the use of such blind holes for receiving pins helps to maintain the pins within the spacer body.

FIGS. 14A-14D illustrate different views of an alternative multi-piece allograft spacer to be used with the lower profile plates discussed above according to some embodiments. The multi-piece allograft spacer 510 can be formed of a left member 536 and a right member 538 that are connected together in series or side-by-side (e.g., laterally) via one or more pins 575. The left member 536 and the right member 538 each include cut-out portions that help form a graft opening 520 in the spacer 510.

The left member 536 can include upper and lower surfaces having bone engagement surfaces (e.g., ridges, teeth, ribs). In addition, the left member 536 further includes a right interfacing surface 546. The right member 538 can also include upper and lower surfaces having bone engagement surfaces (e.g., ridges, teeth, ribs). In addition, the right member 538 further includes a left interfacing surface 548. In some embodiments, the left member 536 can include one or more holes 562, while the right member 538 can include one or more holes 564 which align with the one or more holes 562 of the left member. The aligned holes are configured to receive one or more pins 575 to keep the left and right members of the allograft spacer together.

As shown best in FIG. 14A, the right interfacing surface 546 of the left member 536 is directly engaged with the left interfacing surface 548 of the right member 538. While the right interfacing surface 546 and the left interfacing surface 548 can be flat-on-flat, as both surfaces are planar, in some embodiments (as shown in FIG. 14A), the interface between the two surfaces is at an angle relative to the holes for receiving the pins 575. In other words, the pins 575 are received at an angle to the interface between the left member 536 and the right member 538. In addition, as shown in FIG. 14B, holes 562 and 564 need not go through the entirety of their respective members. In other words, one or more of the holes (e.g., holes 562, 564 or combined) can be blind holes, whereby the holes do not go through the left and right surfaces of the lateral implants.

By having multi-piece allograft spacers that are either stacked or aligned side-by-side, it is possible to have spacers of increased height and width. While the embodiments herein show two piece spacers, one skilled in the art will appreciate that three or more members can be combined to form multi-piece allograft spacers for use with any of the plate members described above.

It will be apparent to those skilled in the art that various modifications and variations can be made in the present invention without departing from the scope of the invention. Moreover, the improved bone screw assemblies and related methods of use need not feature all of the objects, advantages, features and aspects discussed above. Thus, for example, those skilled in the art will recognize that the invention can be embodied or carried out in a manner that achieves or optimizes one advantage or a group of advantages as taught herein without necessarily achieving other objects or advantages as may be taught or suggested herein. In addition, while a number of variations of the invention have been shown and described in detail, other modifications and methods of use, which are within the scope of this invention, will be readily apparent to those of skill in the art based upon this disclosure. It is contemplated that various combinations or subcombinations of these specific features and aspects of embodiments may be made and still fall within the scope of the invention. Accordingly, it should be understood that various features and aspects of the disclosed embodiments can be combined with or substituted for one another in order to form varying modes of the discussed bone screw assemblies. Thus, it is intended that the present invention cover the modifications and variations of this invention provided that they come within the scope of the appended claims or their equivalents.

## Claims

1. A spinal system (5) comprising:
a spacer (10) for inserting into an intervertebral space, the spacer (10) including:
an upper surface (12),
a lower surface (14), and
an opening (20) that extends between the upper surface (12) to the lower surface (14), wherein the spacer (10) further includes a tapered leading end (22), ;
a plate (50) for abutting the spacer (10), the plate (50) including:
a plate body (50),
a first opening (52) formed in the plate body (50) for receiving a first bone screw,
wherein the first opening (52) is angled in an upward direction;
a second opening (54) formed in the plate body (50) for receiving a second bone screw, wherein the second opening (54) is angled in a downward direction;
a set screw (56) for preventing back-out of both the first and the second bone screws, wherein the set screw (56) has a first position whereby the first and second bone screws can be inserted past the set screw (56) and into the first and second openings (52, 54) and a second position following rotation of the set screw (56) whereby the first and second bone screws are prevented from backing out by the set screw (56), and
a pair of extensions (70) that extend from the plate body (50), wherein the extensions (70) are configured to engage the spacer (10),
wherein each extension (70) extends laterally from the plate body (10) and
includes a window (72) that extends along a length of the extension (70) and wherein each of the windows (72) receives a hump area (26) of the spacer to provide a locking mechanism between the spacer (10) and the plate (50);
a first bone screw for inserting into the first opening (52) in the plate body (50), wherein the first bone screw is configured to be inserted into a first vertebral body; and
a second bone screw for inserting into the second opening (54) in the plate body (50),
wherein the second bone screw is configured to be inserted into a second vertebral body different from the first vertebral body.

2. The system of claim 1, wherein the spacer (10) is a C-shaped spacer.

3. The system of claim 1, wherein the spacer (10) includes a first side arm (13) and a second side arm (13), and the first side arm (13) comprises an upper chamfer (18) formed on the upper surface (12) and the second side arm (13) comprises a lower chamfer (19) on the lower surface (14).

4. The system of claim 1, wherein the pair of extensions (70) that extend from the plate body (50) extend around the outer surface of the spacer (10).

5. The system of claim 4, wherein the pair of extensions (70) include inward protrusions (71) for inserting into outer sidewalls of the spacer (10).

6. The system of claim 5, wherein the spacer (10) includes a pair of notches (17) that correspond to the pair of extensions (70) of the plate (50) to form a first locking mechanism between the plate (50) and the spacer (10).

7. The system of claim 1, wherein the opening (20) in the spacer (10) is bounded by at least one convex surface.

8. The system of claim 1, wherein the pair of extensions (70) that extend from the plate body (50) extend within the outer surface of the spacer (10).

9. The system of claim 8, wherein the pair of extensions (70) are part of an enclosed posterior extension that locks the plate (50) to the spacer (10).

10. The system of claim 1, wherein the pair of extensions (70) of the plate (50) comprise lateral extensions that engage an outer surface of the spacer (10).

11. The system of claim 1, wherein the pair of extensions (70) of the plate (50) comprise inward protrusions (71) that extend into notches (17) formed on an outer surface of the spacer (10).

12. The system of claim 1, wherein upon rotation of the set screw (56), the set screw (56) abuts side surfaces of heads of the first and second bone screws.

13. The system of claim 1, wherein the spacer (10) and the plate (50) are independent from one another such that the spacer (10) can be inserted into a desired spinal location prior to abutting the spacer (10) with the plate (50).

14. The system of claim 1, wherein the spacer (10) comprises an allograft spacer or a PEEK spacer.

## Patentansprüche

1. Wirbelsäulensystem (5), umfassend:
- einen Abstandshalter (10) zum Einführen in einen Zwischenwirbelraum, wobei der Abstandshalter (10) umfasst:
- eine obere Fläche (12),
- eine untere Fläche (14) und
- eine Öffnung (20), die sich zwischen der oberen Fläche (12) und der unteren Fläche (14) erstreckt, wobei der Abstandshalter (10) ferner ein konisches vorderes Ende (22) umfasst;
- eine Platte (50) zum Anliegen an den Abstandshalter (10), wobei die Platte (50) umfasst:
- einen Plattenkörper (50),
- eine im Plattenkörper (50) ausgebildete erste Öffnung (52) zur Aufnahme einer ersten Knochenschraube, wobei die erste Öffnung (52) nach oben abgewinkelt ist;
- eine im Plattenkörper (50) ausgebildete zweite Öffnung (54) zur Aufnahme einer zweiten Knochenschraube, wobei die zweite Öffnung (54) nach unten abgewinkelt ist;
- eine Stellschraube (56) zum Verhindern eines Herausdrehens sowohl der ersten als auch der zweiten Knochenschraube, wobei die Stellschraube (56) eine erste Position aufweist, in der die ersten und zweiten Knochenschrauben an der Stellschraube (56) vorbei und in die ersten und zweiten Öffnungen (52, 54) eingeführt werden können, und eine zweite Position nach Drehung der Stellschraube (56) aufweist, in der die ersten und zweiten Knochenschrauben durch die Stellschraube (56) daran gehindert werden, sich herauszudrehen, und
- ein Paar von Verlängerungen (70), die sich vom Plattenkörper (50) erstrecken, wobei die Verlängerungen (70) für den Eingriff mit dem Abstandshalter (10) eingerichtet sind,
- wobei sich jede Verlängerung (70) seitlich vom Plattenkörper (10) erstreckt und ein Fenster (72) umfasst, das sich entlang einer Länge der Verlängerung (70) erstreckt, und
- wobei jedes der Fenster (72) einen Höckerbereich (26) des Abstandshalters aufnimmt, um einen Verriegelungsmechanismus zwischen dem Abstandshalter (10) und der Platte (50) vorzusehen;
- eine erste Knochenschraube zum Einführen in die erste Öffnung (52) im Plattenkörper (50), wobei die erste Knochenschraube eingerichtet ist, um in einen ersten Wirbelkörper eingeführt zu werden; und
- eine zweite Knochenschraube zum Einführen in die zweite Öffnung (54) im Plattenkörper (50), wobei die zweite Knochenschraube eingerichtet ist, um in einen zweiten Wirbelkörper eingeführt zu werden, der vom ersten Wirbelkörper verschieden ist.

2. System nach Anspruch 1, wobei der Abstandshalter (10) ein C-förmiger Abstandshalter ist.

3. System nach Anspruch 1, wobei der Abstandshalter (10) einen ersten Seitenarm (13) und einen zweiten Seitenarm (13) umfasst und der erste Seitenarm (13) eine obere Abschrägung (18) aufweist, die an der oberen Fläche (12) ausgebildet ist, und der zweite Seitenarm (13) eine untere Abschrägung (19) an der unteren Fläche (14) aufweist.

4. System nach Anspruch 1, wobei sich das Paar von Verlängerungen (70), das sich vom Plattenkörper (50) erstreckt, um die Außenfläche des Abstandshalters (10) herum erstreckt.

5. System nach Anspruch 4, wobei das Paar von Verlängerungen (70) nach innen gerichtete Vorsprünge (71) zum Einführen in äußere Seitenwände des Abstandshalters (10) umfasst.

6. System nach Anspruch 5, wobei der Abstandshalter (10) ein Paar von Kerben (17) aufweist, das dem Paar von Verlängerungen (70) der Platte (50) entspricht, um einen ersten Verriegelungsmechanismus zwischen der Platte (50) und dem Abstandshalter (10) zu bilden.

7. System nach Anspruch 1, wobei die Öffnung (20) im Abstandshalter (10) durch mindestens eine konvexe Fläche begrenzt ist.

8. System nach Anspruch 1, wobei sich das Paar von Verlängerungen (70), das sich vom Plattenkörper (50) erstreckt, innerhalb der Außenfläche des Abstandshalters (10) erstreckt.

9. System nach Anspruch 8, wobei das Paar von Verlängerungen (70) Teil einer umschlossenen hinteren Verlängerung ist, die die Platte (50) am Abstandshalter (10) befestigt.

10. System nach Anspruch 1, wobei das Paar von Verlängerungen (70) der Platte (50) seitliche Verlängerungen aufweist, die mit einer Außenfläche des Abstandshalters (10) in Eingriff stehen.

11. System nach Anspruch 1, wobei das Paar von Verlängerungen (70) der Platte (50) nach innen gerichtete Vorsprünge (71) aufweist, die sich in Kerben (17) erstrecken, die an einer Außenfläche des Abstandshalters (10) ausgebildet sind.

12. System nach Anspruch 1, wobei bei Drehung der Stellschraube (56) die Stellschraube (56) an Seitenflächen der Köpfe der ersten und zweiten Knochenschraube anliegt.

13. System nach Anspruch 1, wobei der Abstandshalter (10) und die Platte (50) unabhängig voneinander sind, so dass der Abstandshalter (10) an einer gewünschten Wirbelsäulenstelle eingeführt werden kann, bevor der Abstandshalter (10) an der Platte anliegt (50).

14. System nach Anspruch 1, wobei der Abstandshalter (10) einen Allograft-Abstandshalter oder einen PEEK-Abstandshalter aufweist.

## Revendications

1. Système vertébral (5) comprenant :
un dispositif d'espacement (10) destiné à être inséré dans un espace intervertébral, le dispositif d'espacement (10) comprenant :
une surface supérieure (12),
une surface inférieure (14), et
une ouverture (20) qui s'étend entre la surface supérieure (12) et la surface inférieure (14), dans lequel le dispositif d'espacement (10) comprend en outre une extrémité d'attaque progressivement rétrécie (22) ;
une plaque (50) destinée à venir en butée contre le dispositif d'espacement (10) comprenant :
un corps de plaque (50),
une première ouverture (52) formée dans le corps de plaque (50) pour recevoir une première vis à os, dans lequel la première ouverture (52) est orientée dans une direction ascendante ;
une seconde ouverture (54) formée dans le corps de plaque (50) pour recevoir une seconde vis à os, dans lequel la seconde ouverture (54) est orientée dans une direction descendante ;
une vis de réglage (56) pour empêcher le recul à la fois de la première et de la seconde vis à os, dans lequel la vis de réglage (56) a une première position, dans laquelle les première et seconde vis à os peuvent être insérées au-delà de la vis de réglage (56) et dans les première et seconde ouvertures (52, 54) et une seconde position suite à la rotation de la vis de réglage (56) dans laquelle le recul des première et seconde vis à os est empêché grâce à la vis de réglage (56), et
une paire d'extensions (70) qui s'étend à partir du corps de plaque (50), dans lequel les extensions (70) sont configurées pour mettre en prise le dispositif d'espacement (10),
dans lequel chaque extension (70) s'étend latéralement à partir du corps de plaque (10), et
comprend une fenêtre (72) qui s'étend le long d'une longueur de l'extension (70) et dans lequel chacune des fenêtres (72) reçoit une zone de bossage (26) du dispositif d'espacement pour fournir un mécanisme de blocage entre le dispositif d'espacement (10) et la plaque (50) ;
une première vis à os destinée à être insérée dans la première ouverture (52) dans le corps de plaque (50), dans lequel la première vis à os est configurée pour être insérée dans un premier corps vertébral ; et
une seconde vis à os destinée à être insérée dans la seconde ouverture (54) dans le corps de plaque (50), dans lequel la seconde vis à os est configurée pour être insérée dans un second corps vertébral différent du premier corps vertébral.

2. Système selon la revendication 1, dans lequel le dispositif d'espacement (10) est un dispositif d'espacement en forme de C.

3. Système selon la revendication 1, dans lequel le dispositif d'espacement (10) comprend un premier bras latéral (13) et un second bras latéral (13), et le premier bras latéral (13) comprend un chanfrein supérieur (18) formé sur la surface supérieure (12) et le second bras latéral (13) comprend un chanfrein inférieur (19) sur la surface inférieure (14).

4. Système selon la revendication 1, dans lequel la paire d'extensions (70) qui s'étend à partir du corps de plaque (50), s'étend autour de la surface externe du dispositif d'espacement (10).

5. Système selon la revendication 4, dans lequel la paire d'extensions (70) comprend des saillies vers l'intérieur (71) destinées à s'insérer dans des parois latérales externes du dispositif d'espacement (10).

6. Système selon la revendication 5, dans lequel le dispositif d'espacement (10) comprend une paire d'encoches (17) qui correspond à la paire d'extensions (70) de la plaque (50) afin de former un premier mécanisme de blocage entre la plaque (50) et le dispositif d'espacement (10).

7. Système selon la revendication 1, dans lequel l'ouverture (20) dans le dispositif d'espacement (10) est délimitée par au moins une surface convexe.

8. Système selon la revendication 1, dans lequel la paire d'extensions (70) qui s'étend à partir du corps de plaque (50), s'étend à l'intérieur de la surface externe du dispositif d'espacement (10).

9. Système selon la revendication 8, dans lequel la paire d'extension (70) fait partie d'une extension postérieure close qui bloque la plaque (50) sur le dispositif d'espacement (10).

10. Système selon la revendication 1, dans lequel la paire d'extensions (70) de la plaque (50) comprend des extensions latérales qui mettent en prise une surface externe du dispositif d'espacement (10).

11. Système selon la revendication 1, dans lequel la paire d'extensions (70) de la plaque (50) comprend des saillies vers l'intérieur (71) qui s'étendent dans des encoches (17) formées sur une surface externe du dispositif d'espacement (10).

12. Système selon la revendication 1, dans lequel suite à la rotation de la vis de réglage (56), la vis de réglage (56) vient en butée contre des surfaces latérales des têtes des première et seconde vis à os.

13. Système selon la revendication 1, dans lequel le dispositif d'espacement (10) et la plaque (50) sont indépendants l'un de l'autre de sorte que le dispositif d'espacement (10) peut être inséré dans un emplacement vertébral souhaité avant la venue en butée du dispositif d'espacement (10) avec la plaque (50).

14. Système selon la revendication 1, dans lequel le dispositif d'espacement (10) comprend un dispositif d'espacement d'allogreffe ou un dispositif d'espacement de PEEK.
